# EUROPÄISCHE PATENTSCHRIFT

(11) **EP 0 878 176 B1**
(45) Veröffentlichungstag und Bekanntmachung des Hinweises auf die Patenterteilung: **01.10.2003**
(21) Anmeldenummer: 98107815.7
(22) Anmeldetag: 29.04.1998
(51) Int. Cl.: A61F 2/36

(54) **Schenkelhalsendoprothese für ein künstliches Hüftgelenk**
Femoral neck endoprosthesis for an artificial hip joint
Endoprothèse du col du fémur pour une articulation artificielle de la hanche

(30) Priorität: 16.05.1997 DE 19720493
(43) Veröffentlichungstag der Anmeldung: 18.11.1998
(73) Patentinhaber: ESKA Implants GmbH & Co., 23556 Lübeck (DE)
(72) Erfinder: Grundei, Hans, 23558 Lübeck (DE); Thomas, Wolfram, Prof. Dr., 00135 Rom (IT)
(74) Vertreter: Fuchs Mehler Weiss & Fritzsche

(56) Entgegenhaltungen:
- EP-A- 0 201 407
- EP-A- 0 579 868
- DE-C- 3 600 804
- DE-C- 19 601 340
- FR-A- 2 519 248
- FR-A- 2 721 200
- US-A- 5 433 750

## Beschreibung

Es wird eine Schenkelhalsendoprothese für ein künstliches Hüftgelenk beschrieben, welche eine Alternative zu der Schenkelhalsendoprothese gemäß dem deutschen Patent 196 01 340 ist.

Zur Behandlung eines distruierten, d. h. zerstörten Hüftgelenkskopf, woraus erhebliche Schmerzempfindungen und gravierende funktionelle Einschränkungen des Gelenks resultieren, haben sich im wesentlichen zwei Therapiemöglichkeiten während der letzten Jahrzehnte durchgesetzt:

Zum einen ist es möglich, den Patienten mit einer Orthese zu versorgen, d. h. also mit einem externen Stützapparat. Neben der Tatsache, daß diese Versorgungsmöglichkeit kosmetisch völlig unakzeptabel ist, ist sie auch versorgungstechnisch nur suboptimal.

Als weitere, weitverbreitetere Möglichkeit ist vorgesehen, den Patienten mit einer Endoprothese zu versorgen, namentlich mit einer Hüftstielendoprothese, bei welcher ein Stiel in den zuvor freigeräumten Knochenmarksraums des Femurs eingesetzt wird und dort zementlos oder unter Verwendung eines Zements fixiert wird. An der proximalen Seite weist eine derartige Endoprothese dann die Möglichkeit des Anschlusses einer künstlichen Gelenkkugel vor.

Gerade die letzte Möglichkeit ist stark in der Patentliteratur vertreten. An dieser Stelle sei nur beispielhaft auf die DE 94 12 408 U1 hingewiesen.

Unter den einen oder anderen der in der Patentliteratur vorgeschlagenen Hüftstielendoprothesen gibt es einige Ansätze, die recht vielversprechend sind im Hinblick auf die Langzeitstabilität im Patientenkörper. Früher oder später jedoch, beispielsweise nach 10 bis 15 Jahren, ist zu beobachten, daß die Endoprothese einem Verschleiß unterworfen ist, welcher zu der Notwendigkeit führt, in einem Revisionseingriff die implantierte Endoprothese zu entfernen und durch eine neue zu ersetzen. Dies ist freilich nicht ganz unproblematisch, da durch die Resektion und Ausräumung des Knochenmarkraumes ein nicht unerheblicher Anteil natürlichen Knochenmaterials bei der Erstimplantation entfernt worden ist. Das einmal entfernte Material fehlt dann unter Umständen bei einem Revisionseingriff. Dies kommt insbesondere dann zum Tragen, wenn die Patienten relativ jung sind, bei denen also von vorn herein davon auszugehen ist, daß sie im Laufe ihres Lebens mindestens einem Revisionseingriff unterworfen werden müssen.

Eine Endoprothese, die mit einer geringfügigeren Resektion natürlichen Knochenmaterials auskommt als die bekannten Hüftstielendoprothesen ist bekannt geworden aus der DE 27 24 234 C2. Das darin beschriebene Prothesenteil wird im oberen Bereich eines Femurs, aber unterhalb des Trochanter minors implantiert, ohne daß ein Stiel in den Knochenmarksraum reichen würde. Diese auch als Druckscheibenprothese bezeichnete Prothese greift im wesentlichen mit einer proximalen Druckplatte über die Kortikalis des resezierten Femurs im Bereich des entfernten Hüftgelenkkopfes. Sie wird mit einer Druckplatte, die lateral am Femur anliegt, verspannt, derart, daß alle mechanischen Kräfte zwischen der Endoprothese und dem Femur direkt in die Kortikalschicht des Femurs eingeleitet werden, wodurch eine als unzulässig empfundene mechanische Beanspruchung der Spongiosa vermieden werden soll. Lediglich eine geringe Menge von Knochenzement ist für die Arretierung der Druckscheibe im proximalen Bereich notwendig.

Der Philosophie dieser Prothese liegt - wie ausgeführt - die Annahme zugrunde, daß die Spongiosa des Femurs möglichst wenig beansprucht werden soll. Dies wird erkauft durch eine extreme Belastung der Kortikalis des Femurs, indem nämlich sämtliche Kräfte darauf abgelastet und darin eingeleitet werden. Dies entspricht nach heutiger Erkenntnis in keiner Weise den natürlichen Belastungsverhältnissen.

Eine ganz ähnlich wirkende Prothese zeigt im übrigen die WO 89/11837, bei der die Funktion der proximalen Druckplatte gemäß der vorerwähnten Druckschrift wahrgenommen wird durch den speziell ausgebildeten Hüftgelenkkopf, der innenseitig mit einer Ausnehmung versehen ist, an der sich innenseitig der resizierte Femurstumpf unter Druckerzeugung anlegt.

Zwei Prothesen, bei denen augenscheinlich die Hauptlast von der Spongiosa des Femurknochens aufgenommen werden soll, sind bekanntgeworden aus der FR 26 26 169 A1 und der FR 26 74 122 A1. Bei der erstgenannten Druckschrift der beiden ist vorgesehen, einen proximal einen Steckkonus aufweisenden Gewindebolzen in die Spongiosa einzuschrauben. Dies dürfte innerhalb kürzester Zeit zu schweren Instabilitätsproblemen führen. Bei der zweitgenannten Druckschrift der beiden wird eine einen Steckkonus tragende Platte mittels einer Reihe von Knochenschrauben befestigt, wobei die Knochenschrauben in die Spongiosa reichen. Auch hieraus ergeben sich ernsthafte Stabilitätsfragen, da die Spongiosa von Natur aus im Verhältnis zur Kortikalis des Femurknochens punktuell nur gering belastbar ist.

Vor diesem Hintergrund ist es daher die Aufgabe der vorliegenden Erfindung, eine Schenkelhalsendoprothese für ein künstliches Hüftgelenk anzugeben, die sich hinsichtlich der Krafteinleitung in den Femur bei nur relativ geringfügig notwendig werdenden Resektionen von natürlichen Knochenmaterial den natürlichen Verhältnissen erheblich besser anpaßt als die vorerwähnte Prothese.

Es soll also eine Schenkelhalsendoprothese angegeben werden, die sich hinsichtlich der Krafteinleitung in den Femur bei nur relativ geringfügig notwendig werdenden Resektion von natürlichem Knochenmaterial den natürlichen Verhältnissen erheblich besser anpaßt als bekannte Prothesen.

Zur Lösung der Aufgabe wird vorgesehen, daß die Schenkelhalsendoprothese aufweist:
- eine zementlos im oberen Bereich eines Femurs unterhalb des Trochanter majors implantierbare Hülse, mit deren proximalem Ende ein Adapter zur Aufnahme einer künstlichen Gelenkkugel verbindbar ist, wobei die Hülsenaußenseite zumindest teilweise mit einer offenmaschigen dreidimensionalen Raumnetzstruktur belegt ist, sowie
- ein nach caudal gebogenes, als Stielende ausgebildetes Hülsenende, welches zumindest teilweise mit einer offenmaschigen dreidimensionalen Raumnetzstruktur belegt ist, wobei das Hülsenende eine nach caudal ausgerichtete Stufe in einem Bereich des Übergangs zum Stielende aufweist.

Im Unterschied zum deutschen Patent 196 01 340 ist erfindungsgemäß keine Zugplatte vorgesehen, welche zusammen mit einer Zugschraube wirkt, welche die Kortikalis des Femurknochens durchdringt. Diese Durchdringung führt zu einer Schwächung des Femurknochens.

Viel mehr ist vorliegend das abgebogene Ende der Hülse jenes Teil, welches sich lateral an die kortikalisierte Seite der natürlichen Spongiosa legt. Das erfindungsgemäße Implantat ist also "freifliegend" in der Spongiosa des Schenkelhalses zu verankern, wohingegen das Implantat gemäß dem deutschen Patent 196 01 340 einen Fest- oder Drehpunkt in Form der Zugplatte aufweist.

Vorliegend ist das Ergebnis, daß der Femurknochen von extern her in keiner Weise geschwächt wird. Der Aufbau ist im übrigen einfacher als jener gemäß dem deutschen Patent 196 01 340. Die Ankopplung einer künstlichen Gelenkkugel erfolgt in gleicher Weise mit einem entsprechenden Adapter.

Weitere vorteilhafte Weiterbildungen ergeben sich aus den Unteransprüchen.

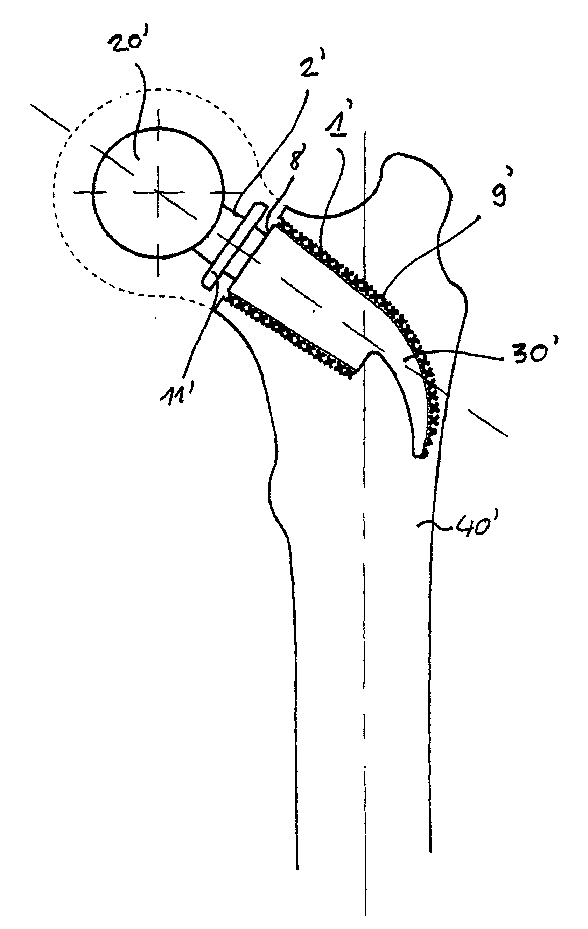

Die Erfindung wird anhand eines Ausführungsbeispieles gemäß der einzigen Zeichnungsfigur näher erläutert.

Vorliegend weist die Hülse 1' einen ähnlichen Verlauf auf wie die Hülse gemäß dem deutschen Patent 196 01 340. Im distalen Bereich jedoch geht die Hülse 1' über in ein nach caudal, d.h. nach unten gebogenes Stielende 30', wobei die Krümmung derart bemessen ist, daß das äußerste Ende des Stielendes 30' im lateralen Bereich des Femurinneren zu liegen kommt.

Die Hülse 1' ist ebenso wie das Stielende 30' mit einer offenmaschigen dreidimensionalen Raumnetzstruktur 9' belegt. Die Ankopplung einer künstlichen Gelenkkugel 20' erfolgt mit dem als Doppelkonus ausgebildeten Adapter 2' in gleicher Weise wie dies im deutschen Patent 196 01 340 vorgesehen ist.

Wie deutlich erkennbar ist, durchdringt kein Teil des Implantates die Kortikalis des Femurknochen 40', so daß keinerlei Schwächung des Knochenmaterials in diesem Bereich stattfindet. In den Fällen, in denen die erfindungsgemäße Schenkelhalsendoprothese eingesetzt werden kann, ist ein stabilerer Femur die Folge.

## Patentansprüche

1. Schenkelhalsendoprothese für ein künstliches Hüftgelenk, aufweisend
- eine zementlos im oberen Bereich eines Femurs (40') unterhalb des Trochanter majors implantierbare Hülse (1'), mit deren proximalem Ende (8') ein Adapter (2') zur Aufnahme einer künstlichen Gelenkkugel (20') verbindbar ist, wobei die Hülsenaußenseite zumindest teilweise mit einer offenmaschigen dreidimensionalen Raumnetzstruktur (9') belegt ist, sowie
- ein nach caudal gebogenes, als Stielende (30') ausgebildetes Hülsenende, welches zumindest teilweise mit einer offenmaschigen dreidimensionalen Raumnetzstruktur (9') belegt ist, wobei das Hülsenende eine nach caudal ausgerichtete Stufe in einem Bereich des Übergangs zum Stielende aufweist.

2. Schenkelhalsendoprothese nach Anspruch 1, bei der die Hülse (1') von ihrem proximalen Ende (8') bis zum Ansatz des gebogenen Stielendes (30') konisch sich verjüngend ausgebildet ist.

3. Schenkelhalsendoprothese nach Anspruch 1 oder 2, bei der die Raumnetzstruktur (9') auf den nach caudal und nach kranial weisenden Hülsenaußenseiten grobmaschig mit Maschenweite zwischen 2 bis 6 mm ausgebildet ist.

4. Schenkelhalsendoprothese nach einem der Ansprüche 1 bis 3, bei der die Raumnetzstruktur (9') in Richtung ventral und dorsal auf der Hülsenaußenseite feinmaschig mit Maschenweiten zwischen 1 bis 2,5 mm ausgebildet ist.

5. Schenkelhalsendoprothese nach einem der Ansprüche 1 bis 4, bei der der Adapter (2') für die Gelenkkugel (20') im wesentlichen als Doppelsteckkonus ausgebildet ist mit einem an den beiden Konusbasen umlaufenden Flansch (11'), wobei im proximalen Bereich der Hülse (1') eine dem einen Steckkonus entsprechend ausgebildete konische Steckhülse vorgesehen ist.

6. Schenkelhalsendoprothese nach einem der Ansprüche 1 bis 5, bei der die nach außen weisenden Flächen des Flansches (11') des Adapters (2') mit einer offenmaschigen dreidimensionalen Raumnetzstruktur belegt sind.

## Claims

1. Femoral neck endoprosthesis for an artificial hip joint having
- a sleeve (1') which can be implanted without cement in the upper region of a femur (40') below the trochanter major, with the proximal end (8') of which sleeve (1') an adapter (2') can be connected to receive an artificial joint head (20'), wherein the outer side of the sleeve is covered at least partly with an open-meshed three-dimensional spatial network structure (9'), as well as
- a sleeve end bent in caudal direction and designed as a stem end (30'), which sleeve end is covered at least partly by an open-meshed three-dimensional spatial network structure (9'), wherein the sleeve end has a step aligned in caudal direction in a region of the transition to the stem end.

2. Femoral neck endoprosthesis according to claim 1, in which the sleeve (1') is designed to be conically tapering from its proximal end (8') as far as the shoulder of the bent stem end (30').

3. Femoral neck endoprosthesis according to claim 1 or 2, in which the spatial network structure (9') is designed to be coarse-meshed having mesh width between 2 to 6 mm on the outer sides of the sleeve pointing in caudal direction and in cranial direction.

4. Femoral neck endoprosthesis according to one of claims 1 to 3, in which the spatial network structure (9') is designed to be fine-meshed having mesh widths between 1 to 2.5 mm in the direction ventrally and dorsally on the outer side of the sleeve.

5. Femoral neck endoprosthesis according to one of claims 1 to 4, in which the adapter (2') for the joint head (20') is designed essentially as a double plug-in cone having a flange (11') running around the two cone bases, wherein a conical plug-in sleeve designed to correspond to the one plug-in cone is provided in the proximal region of the sleeve (1').

6. Femoral neck endoprosthesis according to one of claims 1 to 5, in which the surfaces pointing outwards of the flange (11') of the adapter (2') are covered with an open-meshed three-dimensional spatial network structure.

## Revendications

1. Endoprothèse du col du fémur pour une articulation artificielle de la hanche, comprenant
- une gaine (1'), qui peut être implantée sans ciment dans la région supérieure d'un fémur (40') en dessous du grand trochanter et dont l'extrémité proximale (8') peut être accouplée à un adaptateur (2') destiné à recevoir un cotyle (20') artificiel, la face extérieure de la gaine étant revêtue au moins en partie d'une structure réticulaire (9') tridimensionnelle à mailles ouvertes, ainsi que
- une extrémité de gaine courbée vers la partie caudale et conçue sous forme d'extrémité de tige (30'), qui est revêtue au moins en partie d'une structure réticulaire (9') tridimensionnelle à mailles ouvertes, l'extrémité de la gaine étant munie d'un décrochement orienté vers la partie caudale dans une zone de transition avec l'extrémité de la tige.

2. Endoprothèse du col du fémur selon la revendication 1, dans laquelle la gaine (1') est conçue en se rétrécissant en forme de cône à partir de son extrémité proximale (8') jusqu'au début de l'extrémité courbe (30') de la tige.

3. Endoprothèse du col du fémur selon la revendication 1 ou 2, dans laquelle la structure réticulaire (9') est réalisée avec des mailles larges, à savoir une largeur de mailles comprise entre 2 à 6 mm, sur les faces extérieures de la gaine, orientées vers la partie caudale et la partie crâniale.

4. Endoprothèse du col du fémur selon l'une quelconque des revendications 1 à 3, dans laquelle la structure réticulaire (9') est réalisée avec des mailles fines, à savoir une largeur de mailles comprise entre 1 à 2,5 mm, sur la face extérieure de la gaine en direction de la partie ventrale et de la partie dorsale.

5. Endoprothèse du col du fémur selon l'une quelconque des revendications 1 à 4, dans laquelle l'adaptateur (2') pour le cotyle (20') artificiel est conçu sensiblement en forme de double cône à enficher, avec une collerette (11') périphérique sur les deux bases du cône, une gaine à enficher conique, réalisée de manière correspondante au cône à enficher, étant prévue dans la partie proximale de la gaine (1').

6. Endoprothèse du col du fémur selon l'une quelconque xades revendications 1 à 5, dans laquelle les surfaces de la collerette (11') de l'adaptateur (2'), orientées vers l'extérieur, sont couvertes par une structure réticulaire tridimensionnelle à mailles ouvertes.
